# EUROPEAN PATENT SPECIFICATION

(11) **EP 2 055 343 B1**
(45) Date of publication and mention of the grant of the patent: **28.11.2012**
(21) Application number: 08253499.1
(22) Date of filing: 28.10.2008
(51) Int. Cl.: A61M 25/00, A61M 25/10, A61L 27/00

(54) **Medical device including a thin metallic film component attached to a polymeric component and associated methods**
Medizinische Vorrichtung mit einer Dünnmetallfilmkomponente, die an einer polymerischen Komponente befestigt ist und zugehörige Verfahren
Dispositif médical incluant un composant de film métallique mince fixé à un composant polymère et procédés associés

(30) Priority: 29.10.2007 US 983484 P; 05.09.2008 US 205838
(43) Date of publication of application: 06.05.2009
(73) Proprietor: Cordis Corporation, Miami Lakes, FL 33014 (US)
(72) Inventor: Velasco, Regina, Fremont California 94536 (US); Bonsignore, Craig, Pleasanton California 94566 (US)
(74) Representative: Small, Gary James

(56) References cited:
- US-A1- 2001 039 446
- US-A1- 2003 074 016
- US-A1- 2006 182 907
- US-A1- 2007 061 006

## Description

### BACKGROUND OF THE INVENTION

Field of the Invention

The present invention relates, in general, to medical devices and, in particular, to medical devices that include a thin metallic film component attached to polymeric component and associated methods.

Description of Related Art

A variety of medical devices (e.g. angioplasty devices, catheters and stents) include both thin metallic film components and polymeric components. For example, angioplasty devices can include a thin metallic film balloon and a tubular polymeric catheter.

US 2006/0182907 discloses a medical device comprising a medical device component, a medical device polymeric component abutting the medical device component and a heat shrink covering a portion of each of the medical device component and the medical device polymeric component. The medical device component and the medical device polymeric component are joined one to the other by a weld between the two components. A method is also disclosed in which a medical device polymeric component is abutted against a medical device component. A heat shrink is then applied to cover a portion of each of the medical device component and the medical device polymeric component. Heat is then applied such that a weld is formed between the medical device component and the medical device polymeric component thereby joining the one to the other.

US 2007/0061006 discloses the creation of a shape memory film by chemical vapour deposition, which film can be used to make such things as stents, stent covers, grafts, filter membranes and angioplasty balloons. The films may be metallic. The films may be patterned to include void spaces or fenestrations which may be dimensioned and patterned to impart geometric deformability, may provide openings to permit elution of a drug therethrough, or provide wells into which a drug may be placed for elution.

US 2004/0062890 discloses a catheter balloon having improved balloon bonding. A balloon is contructed from an inner and outer layer which may be fusion bonded together. The balloon may be covered at its ends by a polymer shean or suchline which may be fusion bonded to the balloon.

US 2001/0039446 discloses an encapsulated intraluminal stent-graft comprising an intraluminal stent of shape memory alloy encapsulated in a substantially monolithic covering. In an embodiment an at least partially Unsintered interlayer is interdisposed between inner and outer layers of the covering and adjacent the stent.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings, in which like numerals indicate like elements, of which:
FIG. 1 is a simplified flow chart depicting stages in a method according to an example of the present method;
FIG. 2 is a simplified cross-sectional side view depiction of a thin metallic film angioplasty balloon and associated polymeric tubular catheter relevant to embodiments of the present invention;
FIGs. 3A and 3B are simplified cross-sectional depictions of a portion of a thin metallic film angioplasty balloon and a portion of an associated polymeric tubular catheter prior to the application of heat and/or pressure during a method according to an embodiment of the present invention;
FIGs. 4A and 4B are a simplified depictions of a portion of a thin metallic film angioplasty balloon and a portion of an associated polymeric tubular catheter following the application of heat and/or pressure during a method according to an embodiment of the present invention; and
FIG. 5 is an angioplasty balloon and attached polymeric catheter according to an embodiment of the present invention.

### DETAILED DESCRIPTION OF ILLUSTRATIVE EMBODIMENTS

The following detailed description should be read with reference to the drawings, in which like elements in different drawings are identically numbered. Certain drawings, which are not necessarily to scale, depict selected exemplary embodiments for the purpose of explanation only and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

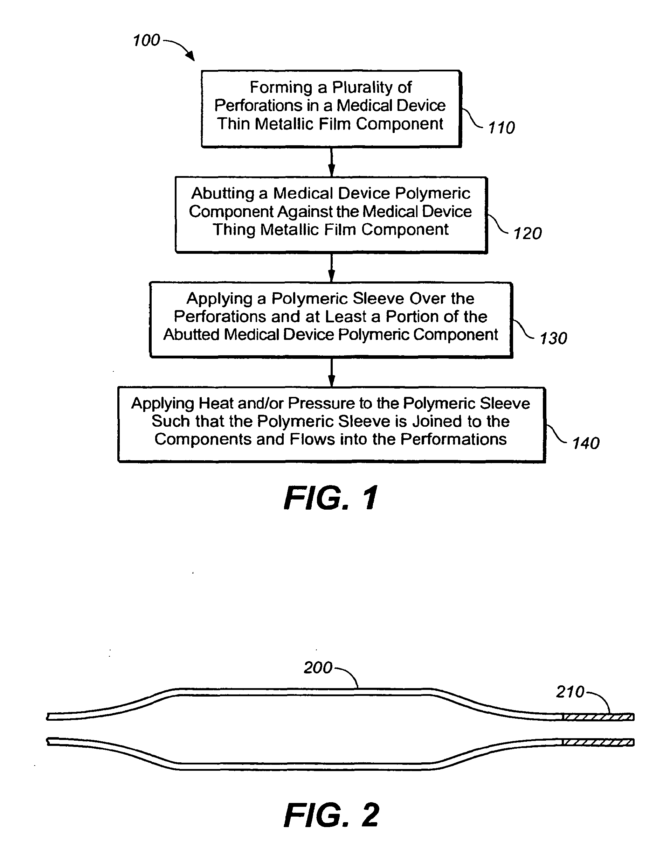

FIG. 1 is a flow diagram depicting stages in method 100 for attaching a medical device thin metallic film component to a medical device polymeric component according to an exemplary process. The medical device thin metallic film component and medical device polymeric component can be any suitable medical device components known to one skilled in the art including, for example, stent components and angioplasty components.

For illustrative purposes only, method 100 will be described with respect to a medical device thin metallic film angioplasty balloon and a medical device tubular polymeric catheter associated with the balloon. FIG. 2 is a simplified cross-sectional side view depiction of a thin metallic film angioplasty balloon 200 and associated polymeric tubular catheter 210 relevant to embodiments of the present invention.

Referring to FIG. 1, method 100 includes, at step 110, forming a plurality of perforations in the medical device thin metallic film component. As previously mentioned, the medical device thin metallic film component can be any suitable medical device thin metallic component including, for example, those formed of aluminum, chromium, cobalt, nickel, niobium, silver, gold, magnesium, manganese, molybdenum, palladium, platinum, scandium, tantalum, titanium, vanadium, zirconium, and combinations thereof such as stainless steel and nitinol. The thickness of the medical device thin metallic film is, for example, in the range of 0,0005 mm (0.5 microns) to 0,02 mm (20 microns).

Once apprised of the present disclosure, a suitable number and size for the perforations can be readily determined by one skilled in the art. A typical non-limiting range for the diameter of circular perorations is 0,005 mm to 0,05 mm (5 microns to 50 microns). Moreover, the perforations can be any suitable shape including, for example, circular, rectangular, oval, and serpentine. A non-limiting example of the number of perforation is a number in the range of 1 perforation to 40 perforations.

The perforations can be formed using any suitable conventional technique including, for example, laser-based and etching perforation techniques.

Next, at step 120, the medical device polymeric component is operatively abutted against the medical device thin film metallic component. The medical device polymeric component can be relatively thin, (having, for example, a thickness in the range of 0,0025 mm to 0,003 mm (2.5 microns to 3.0 microns)). Such relatively thin thicknesses of medical device polymeric components are not readily adhered to medical device thin metallic film components using conventional adhesives but can be securely adhered using methods according to the present invention. Moreover, methods according to the present example provide a medical device with a relatively low profile despite the presence of an attachment region medical device polymeric component.

Subsequently a polymeric sleeve is applied over the perforations and over at least a portion of the abutted medical device polymeric component, as set forth in step 130 of FIG. 1. A typical range for the thickness of the polymeric sleeve is in the range of 0,00254 mm to 0,00732 mm (2.54 µm to 76.2 µm (0.0001 inches to 0.003 inches)). Suitable polymers for use in the polymeric sleeve include, for example, nylon and Pebax. The polymeric sleeve can overlap the medical device thin metallic film and the medical device polymeric component by any suitable amount including, for example, a length in the range of 1 mm to 10mm. For medical devices that require flexibility, such as those that include an angioplasty balloon and tubular polymeric catheter, the polymeric sleeve can beneficially be formed of a flexible polymer with a Durameter value in the range of 10 Durameter to 40 Durameter. Once apprised of the present disclosure, one skilled in the art can readily select flexible polymeric materials suitable for use is exemplary devices.

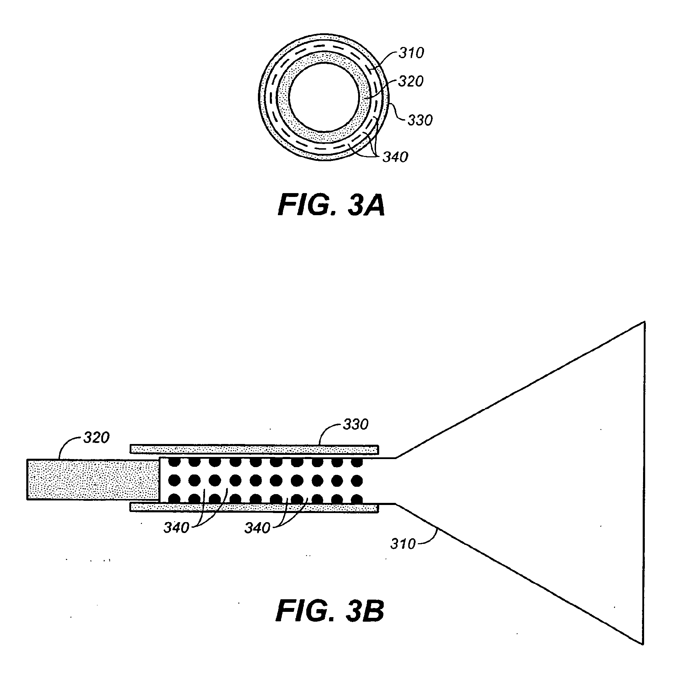

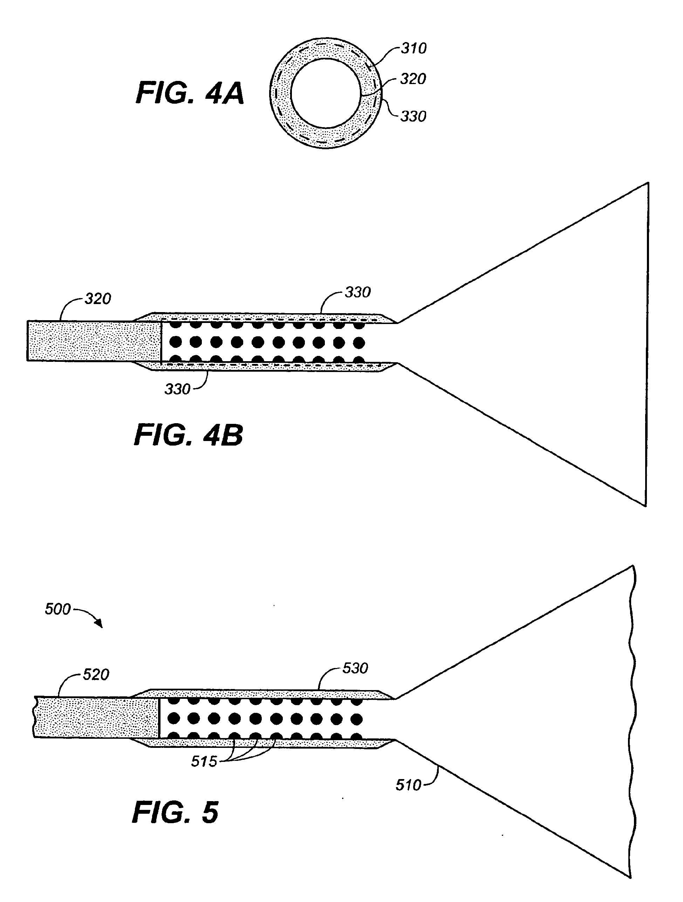

The polymeric sleeve can be applied using any suitable technique including mechanical application of a preformed sleeve or the deposition of a polymeric material to form a sleeve on the surface of the medical device thin metallic film component and the medical device polymeric component. Moreover the term "sleeve" refers generally to a covering or layer and also to a sheath (as illustrated in FIGs. 3A, 3B, 4A and 4B).

FIGs. 3A and 3B are simplified depictions of a portion of a thin metallic film angioplasty balloon 310, a portion of an associated polymeric tubular catheter 320, and a polymeric sleeve 330 prior to the application of heal and/or pressure during a method according to an embodiment of the present invention as claimed in claim 1. FIG. 3 also depicts a plurality of perforations 340 that were previously formed in thin metallic film angioplasty balloon. FIGs. 3A and 3B, therefore, serve to generally illustrate method 100 following step 130.

Referring again to FIG. 1, at least one of heat and pressure is subsequently applied to the polymer sleeve such that the polymer sleeve is joined to the medical device metallic film and the portion of the abutted medical device polymeric component and such that the polymer sleeve flows into the perforations. This joining results in the medical device thin metallic film component being securely attached to the medical device polymeric component. See step 140 of FIG. 1., the polymeric tubular catheter is positioned such that it extended into the thin metallic film angioplasty balloon and underlined the plurality of perforations. The polymeric sleeve joins with the portion(s) of the polymeric tubular catheter that underlies the perforations during the application of heat and/or pressure.

Any suitable temperature and pressure can be employed in step 140. For example, a temperature equivalent to the glass transition temperature of the polymeric sleeve and medical device polymeric component can be employed. Application of suitable pressure will result in the polymeric sleeve flowing into (intruding into) the perforations and becoming joined with the medical device polymeric component. In this respect, it is beneficial to employ a polymeric sleeve that is made of the same polymeric material as the medical device polymeric component. The temperature applied in step 240 can, for example, be in the range of 65.6°C to 121.1°C (150F to 250F).

FIGs. 4A and 4B are simplified depictions of a portion of a thin metallic film angioplasty balloon 310, a portion of an associated polymeric tubular catheter 320 and a polymeric sleeve 330 following the application of heat and/or pressure during a method according to an embodiment of the present invention. FIGs. 4A and 4B, therefore, serve to generally illustrate method 100 following step 140.

FIG. 5 is simplified cross-sectional view of a medical device 500 (depicted as an angioplasty balloon and attached polymeric catheter according to an embodiment of the present invention. Medical device 500 includes a thin metallic film angioplasty balloon 510, with perforations 515 therethrough, and a polymeric tubular catheter 520 abutting the thin metallic film angioplasty balloon The medical device also includes a polymeric sleeve 530 covering the perforations and at least a portion of the polymeric tubular catheter abutting the thin metallic film angioplasty balloon. Moreover, the polymeric sleeve intrudes into the perforations and joins the thin metallic film angioplasty balloon to the polymeric tubular catheter.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that devices and methods within the scope of these claims and their equivalents be covered thereby.

## Claims

1. A medical device comprising:
a thin metallic film angioplasty balloon including a proximal end and a distal end, wherein the proximal end includes a plurality of perforations through the thickness of the thin metallic film angioplasty balloon;
a tubular polymeric catheter including a proximal end and a distal end, wherein the distal end of the tubular polymeric catheter is abutting the thin metallic film angioplasty balloon on the proximal end of the thin metallic film angioplasty alloon; and
a polymeric sleeve covering the perforations on the proximal end of the thin metallic film angioplasty balloon and at least a portion of the tubular polymeric catheter abutting the thin metallic film angioplasty balloon, the polymeric sleeve intruding into the perforations through the thickness of the thin metallic film angioplasty balloon and joining the thin metallic film angioplasty balloon to the tubular polymeric catheter.

2. The medical device of claim 1 wherein the tubular thin metallic film angioplasty balloon is made of nitinol.

3. The medical device of claim 1 wherein the tubular polymeric catheter and the polymeric sleeve are formed of the same polymeric material.

4. The medical device of claim 1 wherein the perforations are circular perforations with a diameter in the range of 5 µm to 50 µm.

5. A method for attaching a thin metallic film angioplasty balloon to a tubular polymeric catheter comprising:
forming a plurality of perforations in a proximal end of the thin metallic film angioplasty balloon and through the thickness of the thin methallic film angioplasty balloon;
abutting a distal end of the tubular polymeric catheter against the proximal end of the thin metallic film angioplasty balloon;
applying a polymeric sleeve over the perforations in the proximal end of the thin metallic film angioplasty balloon and at least a portion of the abutted tubular polymeric catheter;
applying at least one of heat and pressure to the polymeric sleeve such that the polymer sleeve is joined to the thin metallic film angioplasty balloon and the portion of the abutted tubular polymeric catheter and such that the polymeric sleeve flows into the perforation and through the thickness of the thin metallic film angioplasty balloon , thereby attaching the thin metallic film angioplasty balloon to the tubular polymeric catheter.

6. The method of claim 5 wherein the thin metallic film angioplasty balloon is made of nitinol.

7. The method of claim 5 wherein the tubular polymeric catheter and the polymeric sleeve are formed of the same polymeric material.

8. The method of claim 5 wherein the forming step forms circular perforations with a diameter in the range of 5 µm to 50 µm,

9. The medical device of claim 1 wherein the distal end of the tubular polymeric catheter coaxially aligns with the proximal end of the thin metallic film angioplasty balloon.

## Patentansprüche

1. Medizinische Vorrichtung, die Folgendes umfaßt:
einen Angioplastie-Ballon aus dünner Metallfolie mit einem proximalen Ende und einem distalen Ende, worin das proximale Ende eine Vielzahl von Perforationen durch die Dicke des Angioplastie-Ballons aus dünner Metallfolie aufweist;
einen röhrenförmigen Polymer-Katheter mit einem proximalen Ende und einem distalen Ende, worin das distale Ende des röhrenförmigen Polymer-Katheters an den Angioplastie-Ballon aus dünner Metallfolie am proximalen Ende des Angioplastie-Ballons aus dünner Metallfolie anstößt; und
eine Polymer-Hülse, welche die Perforationen auf dem proximalen Ende des Angioplastie-Ballons aus dünner Metallfolie und zumindest einen Teil des an den Angioplastie-Ballon aus dünner Metallfolie anstoßenden röhrenförmigen Polymer-Katheters abdeckt, wobei die Polymer-Hülse in die Perforationen durch die Dicke des Angioplastie-Ballons aus dünner Metallfolie eindringt und den Angioplastie-Ballon aus dünner Metallfolie mit dem röhrenförmigen Polymer-Katheter verbindet.

2. Medizinische Vorrichtung nach Anspruch 7., worin der Angioplastie-Ballon aus dünner Metallfolie aus Nitinol besteht.

3. Medizinische Vorrichtung nach Anspruch 1, worin der röhrenförmige Polymer-Katheter und die Polymer-Hülse aus demselben Polymermaterial geformt sind.

4. Medizinische Vorrichtung nach Anspruch 1, worin die Perforationen kreisförmigen Perforationen mit einem Durchmesser im Bereich von 5 µm bis 50 µm sind.

5. Verfahren zur Befestigung eines Angioplastie-Ballons aus dünner Metallfolie an einem röhrenförmigen Polymer-Katheter, das Folgendes umfasst:
Bildung einer Vielzahl von Perforationen in einem proximalen Ende des Angioplastie-Ballons aus dünner Metallfolie und durch die Dicke des Angioplastie-Ballons aus dünner Metallfolie;
Anlegen eines distalen Endes des röhrenförmigen Polymer-Katheters an das proximale Ende des Angioplastie-Ballons aus dünner Metallfolie;
Auflegen einer Polymer-Hülse über die Perforationen im proximalen Ende des Angioplastie-Ballons aus dünner Metallfolie und zumindest einen Teil des angelegten röhrenförmigen Polymer-Katheters;
Aufbringen von zumindest Wärme und/oder Druck auf die Polymer-Hülse, so dass die Polymer-Hülse mit dem Angioplastie-Ballon aus dünner Metallfolie und dem Absahnitt des angelegten röhrenförmigen Polymer-Katheters verbunden wird und so, dass die Polymer-Hülse in die Perforationen und durch die Dicke des Angioplastie-Ballons aus dünner Metallfolie dringt, wodurch der Angioplastie-Ballon aus dünner Metallfolie mit dem röhrenförmigen Polymer-Katheter verbunden wird.

6. Verfahren nach Anspruch 5, worin der Angioplastie-Ballon aus dünner Metallfolie aus Nitinol besteht.

7. Verfahren nach Anspruch 5, worin der röhrenförmige Polymer-Katheter und die Polymer-Hülse aus demselben Polymermaterial geformt sind.

8. Verfahren nach Anspruch 5, worin der Bildungsschritt kreisförmige Perforationen mit einem Durchmesser im Bereich von 5 µm bis 50 pm formt.

9. Medizinische Vorrichtung nach Anspruch 1, worin das distale Ende des röhrenförmigen Polymer-Katheters koaxial mit dem proximalen Ende des Angioplastie-Ballons aus dünner Metallfolie ausgerichtet ist.

## Revendications

1. Dispositif médical, comprenant:
un ballon d'angioplastie en film métallique mince présentant une extrémité proximale et une extrémité distale, dans lequel l'extrémité proximale comporte une pluralité de perforations à travers l'épaisseur du ballon d'angioplastie en film métallique mince;
un cathéter polymère tubulaire présentant une extrémité proximale et une extrémité distale, dans lequel l'extrémité distale du cathéter polymère tubulaire est adjacente au ballon d'angioplastie en film métallique mince sur l'extrémité proximale du ballon d'angioplastie en film métallique mince; et
un manchon polymère qui couvre les perforations sur l'extrémité proximale du ballon d'angioplastie en film métallique mince et au moins une partie du cathéter polymère tubulaire adjacente au ballon d'angioplastie en film métallique mince, le manchon polymère s'introduisant dans les perforations à travers l'épaisseur du ballon d'angioplastie en film métallique mince et joignant le ballon d'angioplastie en film métallique mince au cathéter polymère tubulaire.

2. Dispositif médical selon la revendication 1, dans lequel le ballon d'angioplastie en film métallique mince est constitué de nitinol.

3. Dispositif médical selon la revendication 1, dans lequel le cathéter polymère tubulaire et le manchon polymère sont constitués du même matériau polymère.

4. Dispositif médical selon la revendication 1, dans lequel les perforations sont des perforations circulaires dont le diamètre est compris dans la gamme de 5 µm à 50 µm.

5. Procédé de fixation d'un ballon d'angioplastie en film métallique mince à un cathéter polymère tubulaire, comprenant les étapes suivantes:
former une pluralité de perforations dans une extrémité proximale du ballon d'angioplastie en film métallique mince et à travers l'épaisseur du ballon d'angioplastie en film métallique mince;
amener une extrémité distale du cathéter polymère tubulaire contre l'extrémité proximale du ballon d'angioplastie en film métallique mince;
appliquer un manchon polymère sur les perforations dans l'extrémité proximale du ballon d'angioplastie en film métallique mince et sur au moins une partie du cathéter polymère tubulaire adjacent; et
appliquer au moins soit de la chaleur, soit de la pression au manchon polymère, de telle sorte que le manchon polymère soit joint au ballon d'angioplastie en film métallique mince et à la partie du cathéter polymère tubulaire adjacent, et de telle sorte que le manchon polymère se glisse dans les perforations et à travers l'épaisseur du ballon d'angioplastie en film métallique mince, attachant de ce fait le ballon d'angioplastie en film métallique mince au cathéter polymère tubulaire.

6. Procédé selon la revendication 5, dans lequel le ballon d'angioplastie en film métallique mince est constitué de nitinol.

7. Procédé selon la revendication 5, dans lequel le cathéter polymère tubulaire et le manchon polymère sont constitués du même matériau polymère.

8. Procédé selon la revendication 5, dans lequel l'étape de formation forme des perforations circulaires dont le diamètre est compris dans la gamme de 5 µm à 50 µm.

9. Dispositif médical selon la revendication 1, dans lequel l'extrémité distale du cathéter polymère tubulaire s'aligne coaxialement avec l'extrémité proximale du ballon d'angioplastie en film métallique mince.
